# EUROPÄISCHE PATENTANMELDUNG

(11) **EP 2 810 650 A1**
(43) Veröffentlichungstag der Anmeldung: **10.12.2014**
(21) Anmeldenummer: 13170956.0
(22) Anmeldetag: 06.06.2013
(51) Int. Cl.: A61K 36/185, A61K 36/25, A61K 36/53

(54) **Arzneimittel enthaltend Thymian und Primel oder Thymian und Efeu als PDE-4-Hemmer**

(71) Anmelder: BIONORICA SE, 92318 Neumarkt (DE)
(72) Erfinder: Popp, Michael.A., 91207 Lauf-Heuchling (DE)
(74) Vertreter: Simandi, Claus

(57) **Zusammenfassung**

Die Erfindung betrifft einen Extrakt aus Thymian (*Thymus L.)* in Kombination mit Primel *(Primula veris)* oder Efeu (*Hedera* helix) zur Verwendung als PDE-4-Inhibitor in der Prophylaxe und Behandlung von ausgewählten Erkrankungen und ein entsprechendes Arzneimittel und dessen Verwendung.

## Beschreibung

Die Erfindung betrifft einen Extrakt aus Thymian (*Thymus L.*) in Kombination mit Primel (*Primula veris*) oder Efeu (*Hedera helix*) zur Verwendung als PDE-4-Inhibitor in der Prophylaxe und Behandlung von ausgewählten Erkrankungen und ein entsprechendes Arzneimittel und dessen Verwendung.

Bronchipret® (eingetragene Marke der BIONORICA SE) ist ein bereits bekanntes hochpotentes Arzneimittel - ein Phytopharmakon, welches Thymian in Kombination mit Primel oder Efeu enthält. Bronchipret® wird vorzugsweise in Form von (Film-)Tabletten (Thymian/Primel - Trockenextrakt) und Saft oder Tropfen (Thymian/Efeu) vertrieben. Die Herstellung des Arzneimittels ist bekannt. Die sekretolytische, bronchospasmolytische, hustenreizstillende, antiphlogistische, antibakterielle und antivirale Wirkung ist beschrieben. Ferner ist aus WO2011/147992 der Anmelderin die Eignung von Bronchipret® zur Behandlung von COPD beschrieben.

Weiterhin beschreibt R. van den Hoeven (R. van den Hoeven et al, Study of the effect of the lung function of five Austrian saddle horses suffering recurrent airways obstruction (heaves), The Veterinary Record (2003), 152, 555-557) den Einfluss von Bronchipret® auf die Lungenfunktion beim Pferd.

Ferner beschreibt z.B. Irwin Ziment, dass Bronchipret® besonders wirksam zur Behandlung von Bronchitis ist (Irwin Ziment, Complementary and alternative medicine therapies for chronic obstructive pulmonary disease Focus Altern Complement Ther 2003; 8: 385-91).

Es besteht jedoch ein hohes Bedürfnis neue wirksame Arzneimittel zur Behandlung und Prophylaxe von weiteren Erkrankungen der Atemwege und Lunge bereitzustellen.

Überraschender Weise zeigt Bronchipret®, insbesondere eine pharmazeutische Zusammensetzung bestehend aus Thymian und Efeu oder Thymian und Primel einen inhibitorischen Effekt auf das Targetenzym "PDE-4" in der Lunge eines Säugetieres, insbesondere eines Menschen (Uzunov, P. and Weiss, B.: Separation of multiple molecular forms of cyclic adenosine 3',5'-monophosphate phosphodiesterase in rat cerebellum by polyacrylamide gel electrophoresis. Biochim. Biophys. Acta 284:220-226, 1972). Daher ist Bronchipret® als Phosphodiesterase-4-Inhibitor ("PDE-4-Hemmer") geeignet.

Es gibt 11 verschiedene Phosphodiesterase-Typen (PDE1 bis PDE11). Von den PDE4-Subtypen sind 4 bekannt (PDE4A bis PDE4D). Phosphodiesterase-4-Inhibitoren (auch Phosphodiesterase-IV-Hemmer) sind Substanzen, die spezifisch das Enzym Phosphodiesterase IV hemmen. Die Phosphodiesterase IV bewirkt eine Umwandlung und Abbau des antientzündlich wirkenden Botenstoffs cAMP (cyklisches Adenosinmonophosphat) in AMP. Dadurch dominieren die entzündungsfördernden Stoffe. PDE-4-Inhibitoren erhöhen jedoch vorteilhaft die Konzentration von intrazellulärem cAMP. Zyklisches AMP (cAMP) spielt eine wichtige Rolle bei der Bewältigung von Entzündungsreaktionen. Eine verlängerte Lebenszeit von cAMP vermittelt durch PDE-4 Inhibitoren bewirkt eine anti-inflammatorische Wirkung.

Ein seit 1979 bisher nicht zugelassenes Arzneimittel als Archetyp eines Phosphodiesterase-4-Inhibitors ist der Wirkstoff Rolipram, welcher zur Behandlung von Depression und Multipler Sklerose entwickelt wurde. PDE-4-Inhibitoren wirken jedenfalls entzündungshemmend und wurden u.a. in den Anwendungsgebieten COPD, Asthma bronchiale, Depression und Multiple Sklerose untersucht. Spezifische Inhibitoren der Isoform 4 (PDE4) werden z.B. bei der Behandlung von COPD eingesetzt (z.B. Daxas®). Bislang wurde lediglich ein Wirkstoff als Arzneimittel zugelassen und zwar Roflumilast (Daxas®) für die Behandlung von Asthma und COPD. Die Entwicklung von Cipamfyllin wurde eingestellt.

Daher erweist Bronchipret® einen curativen und pallativen Effekt zur Prophylaxe und Behandlung von Erkrankungen der Atemwege und Lunge ausgewählt aus der Gruppe der durch PDE-4-Inhibitoren verbundenen Erkrankungen.

Die Erfindung betrifft daher ein Arzneimittel enthaltend Thymian und Efeu oder Thymian und Primel, insbesondere eines Extraktes aus Thymian und Efeu oder Thymian und Primel, zur Verwendung in der Prophylaxe und Behandlung von Erkrankungen der Atemwege und Lunge ausgewählt aus der Gruppe der durch PDE-4-Inhibitoren verbundenen Erkrankungen und zwar:
Asthma-Formenkreis ausgewählt aus der Gruppe bestehend aus atopischem Asthma, nicht-atopischem Asthma, allergischem Asthma, atopischem IgE-vermitteltem Bronchialasthma, Bronchialasthma, essentiellem Asthma, intrinsischem Asthma, verursacht durch pathophysiologische Störungen, extrinsischem Asthma, verursacht durch Umweltfaktoren, essentiellem Asthma aus unbekannter oder nicht-offensichtlicher Ursache, nicht-atopischem Asthma, bronchitischem Asthma, emphysematösem Asthma, Belastungs-induziertem Asthma, Allergen-induziertem Asthma, Asthma, das durch kalte Luft induziert wird, berufsbedingtem Asthma, Infektionsasthma, das durch Bakterien- , Pilz-, Protozoen- oder Virusinfektion verursacht wird, nichtallergischem Asthma, beginnendem Asthma, Kurzatmigkeitssyndrom bzw. Keuchen bei Säuglingen bzw. Kleinkindern ("wheezy infant syndrome");
Bronchiolitis, chronischer oder akuter Bronchokonstriktion, Obstruktion der kleinen Luftwege und Emphysem; obstruktiver oder entzündlicher Luftwegeerkrankungen eines beliebigen Typs, einer beliebigen Ätiologie oder Pathogenese, insbesondere eine obstruktive oder entzündliche Luftwegeerkrankung, die ein Mitglied ist, ausgewählt aus der Gruppe, bestehend aus chronischer eosinophiler Pneumonie, Schocklunge (ARDS),
Exazerbation einer Hyperreaktivität der Luftwege als Folge einer anderen Wirkstofftherapie und Luftwegeerkrankung, die mit pulmonalem Hochdruck assoziiert ist,
Bronchitis-Formenkreis ausgewählt aus der Gruppe bestehend aus akuter laryngotrachealer Bronchitis, durch Erdnüsse ausgelöster Bronchitis ("arachidic bronchitis"), Bronchitis, catarrhalis, Kruppbronchitis, trockener Bronchitis, infektiöser asthmatischer Bronchitis, produktiver Bronchitis, Staphylococcus- oder Streptokokken-Bronchitis und Bläschenbronchitis,
akuter Lungenverletzung, Emphysem, posttraumatischer Lungeninsuffizienz;
Bronchiektasie eines beliebigen Typs, einer beliebigen Ätiologie oder Pathogenese, insbesondere Bronchiektasie-Formenkreis ausgewählt aus der Gruppe, bestehend aus zylinderförmiger Bronchiektasie, sackförmigen Bronchiektasen, fusiformer Bronchiektasie, Bronchiolektasie ("capillary bronchiectasis"), cystischer Bronchiektasie, trockener Bronchiektasie und follikulären Bronchiektasen.

Weiterhin betrifft die Erfindung die Verwendung von Thymian und Efeu oder Thymian und Primel oder eines jeweiligen Extraktes davon zur Herstellung eines Arzneimittels zur Prophylaxe und Behandlung von Erkrankungen der Atemwege und Lunge ausgewählt aus der Gruppe der PDE-4 verbundenen Erkrankungen.

Weiterhin ist erfindungsgemäß vorteilhaft, dass Thymian und Efeu oder Thymian und Primel oder eines jeweiligen Extraktes davon eine Mukoziliäre Clearance erlauben. Die Mukoziliäre Clearance ist ein Mechanismus, bei dem sowohl Fremdpartikel als auch Schleim aus Bronchien und Luftröhre nach oben abtransportiert werden. Hierzu sind die Epithelzellen mit Zilien besetzt, die rhythmisch schlagen und den Schleim fortbewegen. Eine eingeschränkte Schlagfrequenz (CBF) führt zum Erlahmen des Abtransports und wird durch Husten kompensiert. Ein Antrieb der Schlagfrequenz kann daher Husten beseitigen. Überraschender Weise zeigen Thymian und Efeu oder Thymian und Primel oder eines jeweiligen Extraktes davon eine vorteilhafte Induktion der ziliären Schlagfrequenz, so dass der curative und pallative Effekt der durch PDE-4-Inhibitoren verbundenen Erkrankungen positiv unterstützt wird.

Im Rahmen dieser Erfindung bezeichnet Erkrankungen der Atemwege und Lunge ausgewählt aus der Gruppe der durch PDE-4-Inhibitoren verbundenen Erkrankungen, solche Erkrankungen wie vorstehend ausgeführt. Die genannten Indikationen samt ihren Ursachen und Ätiologie können z.B. Mutschler, E. et al., Arzneimittelwirkungen, 8. Aufl., wiss. Verl. Ges. mbH, Stuttgart, 2001 oder Pschyrembel, de Gruyter, Berlin, 2012 entnommen werden.

Die genannten erfindungsgemäßen Pflanzen(drogen) für das erfindungsgemäße Arzneimittel können wie üblich für die jeweilige Pflanzendroge aus bevorzugten Pflanzenteilen gewonnen werden, wie Blatt, Wurzel, etc., wie einschlägig beschrieben (siehe z.B. Ph.Eur., Europäisches Arzneibuch). Insbesondere können wässrige und/oder ethanolische Extrakte verwendet werden oder daraus erhältliche Trockenextrakte. Zur Herstellung der erfindungsgemäßen Extrakte wird auf die technischen Lehren, die Gegenstand von EP 1368605B1, EP 0753306B1 sind, verwiesen.

In vorteilhafter und an sich bekannter Weise können die Arzneimittel der vorliegenden Erfindung hergestellt werden und können in Form einer ihrer Anwendung entsprechenden galenischen Formulierung eingesetzt werden.

Die galenischen Formulierungen der erfindungsgemäßen Arzneimittel sind **dadurch gekennzeichnet, dass** die oralen Formulierungen wie Dragees, Tabletten, Filmtabletten, Pulver, Kapseln oder flüssige Verdünnungen, insbesondere Tropfen, Säfte oder Sirupe umfassen.

Bei Einsatz zur topischen Applikation eignen sich insbesondere Sprays, Salben, Emulsionen, Puder, Pulver, flüssige oder feste Präparate für die Inhalation, Kompressen, Tamponaden, Tonsillenpinsellösungen oder Gurgellösungen.

Ferner betrifft die Erfindung eine pharmazeutische Formulierung enthaltend ein erfindungsgemäßes Arzneimittel. Die erfindungsgemäßen Arzneimittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.
Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen, Pasten, Salben, Gele, Cremes, Lotions, Puder und (Nasen)Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.
Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Weitere Vorteile und Merkmale der vorliegenden Erfindung ergeben sich aufgrund der Beschreibung von Ausführungsbeispielen. Die nachfolgenden Beispiele und Figuren dienen zur Erläuterung der Erfindung ohne die Erfindung auf diese Beispiele zu beschränken.

### 1. Hemmung der Phosphodiesterase-4-Aktivität

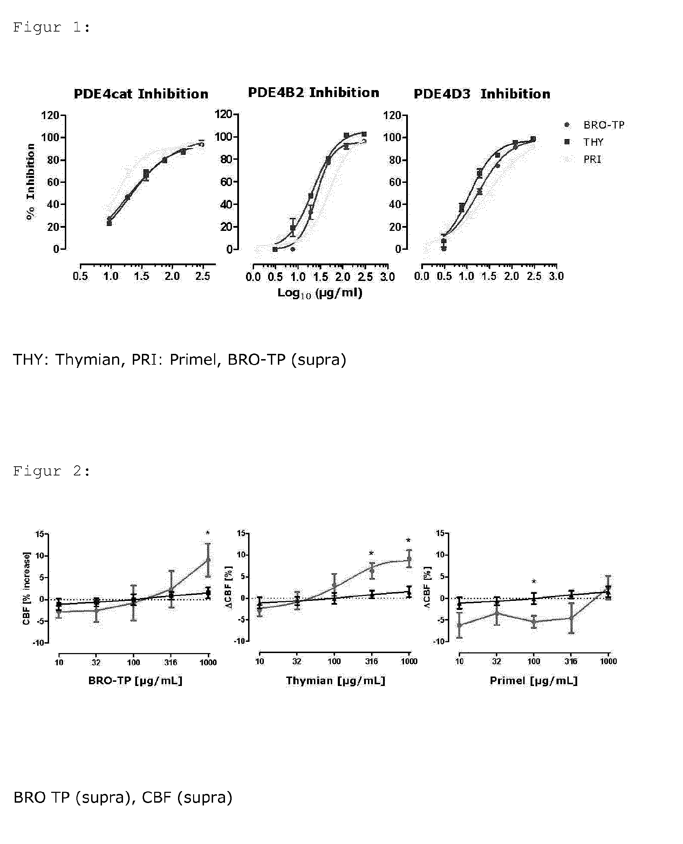

In einem in vitro Standard-Experiment konnte gezeigt werden, dass die Mischung aus Thymian- und Primel-Trockenextrakt ("BRO-TP") in der Lage ist, konzentrationsabhängig die Aktivität der katalytischen Domäne der PDE4 Enzyme zu hemmen Die IC50 lag dabei bei 21 - 26 µg/mL. Ebenfalls die Einzelextrakte hemmten die Aktivität der katalytischen Domäne (Thymian: ca. 20 µg/mL; Primel: 10 - 12 µg/mL). In weiteren Experimenten wurde die spezifische Wirkung auf die PDE4-Subtypen PDE4B und PDE4D untersucht. Auch hier konnte für alle Extrakte inhibitorische Aktivität gezeigt werden: Bei PDE4B2 lagen die IC50-Werte von BRO-TP, Thymian und Primel bei 26, 22 und 51 µg/mL, für PDE4D3 bei 17, 11 und 28 µg/mL (siehe Figur 1).

Methodik: Die katalytische Domäne (PDE4Cat) und die Enzymsubtypen PDE4B2 und -D3 wurden mit 6 Konzentrationen von BRO-TP inkubiert (1,23 - 300 µg/mL) und die katalytische Aktivität in einem radiometrischen Assay bestimmt. Als Positivkontrolle diente der Wirkstoff Rolipram (supra).

### 2. Induktion der ziliären Schlagfrequenz

In einem ex vivo Experiment wurden dünne Lungenschnitte (Precision-Cut Lung Slices - PCLS) mit der Thymian/Primel Extraktmischung und den Einzelextrakten behandelt und die Schlagfrequenz der epitheliären Zilien analysiert. Die Behandlung mit BRO-TP (supra) führte ab 316 µg/mL zu einer Steigerung der CBF. Diese Steigerung war bei 1000 µg/mL signifikant. Thymian induzierte die CBF ab 100 µg/mL, ab 316 µg/mL war dies signifikant. Primel hingegen führte zu keiner Induktion der CBF sondern eher zu einer Inhibition (siehe Figur 2).

Methodik: Aus Meerschweinchenlungen wurden 300 um dicke PCLS gewonnen und in 24-Well-Platten mit Medium überführt. Zum Medium wurden aufsteigende Konzentrationen der Extrakte gegeben (10µg/mL, 32µg/mL, 100µg/mL, 320µg/mL, and 1000µg/mL) und die Schlagfrequenz am Ziliensaum der Atemwege videomikroskopisch analysiert. Zum Vergleich wurde der Einfluss des Vehikels (0,005 - 0,5 % Ethanol) bestimmt. Die maximale Frequenzsteigerung wurde durch Zugabe von Methacholin bestimmt (10 µM).

Es konnte gezeigt werden, dass die anti-inflammatoriche Wirkung der Thymian/Primel Trockenextraktmischung u.a. über eine Hemmung der PDE4 Enzymaktivität vermittelt werden. Außerdem geht von Thymianextrakt eine ex vivo zilienschlagstimulierende Wirkung aus, die auch durch die Extraktmischung hervorgerufen werden kann. Die Ergebnisse wurden in Modellen erhoben, die relevant für die Pathogenese und Therapie der beanspruchten Indikationen sind und liefern damit hinreichend Beweis, dass insbesondere die Kombination von Thymian- und Primel-(Trocken)Extrakt, wie sie in Bronchipret® enthalten ist, auch bei der Behandlung von Patienten wirksam sind.

## Patentansprüche

1. Arzneimittel enthaltend Thymian und Efeu oder Thymian und Primel zur Verwendung als PDE-4 Inhibitor in der Prophylaxe und Behandlung von Erkrankungen ausgewählt aus der Gruppe
Asthma-Formenkreis ausgewählt aus der Gruppe bestehend aus atopischem Asthma, nicht-atopischem Asthma, allergischem Asthma, atopischem IgE-vermitteltem Bronchialasthma, Bronchialasthma, essentiellem Asthma, intrinsischem Asthma, verursacht durch pathophysiologische Störungen, extrinsischem Asthma, verursacht durch Umweltfaktoren, essentiellem Asthma aus unbekannter oder nicht-offensichtlicher Ursache, nicht-atopischem Asthma, bronchitischem Asthma, emphysematösem Asthma, Belastungs-induziertem Asthma, Allergen-induziertem Asthma, Asthma, das durch kalte Luft induziert wird, berufsbedingtem Asthma, Infektionsasthma, das durch Bakterien-, Pilz-, Protozoen- oder Virusinfektion verursacht wird, nichtallergischem Asthma, beginnendem Asthma, Kurzatmigkeitssyndrom bzw. Keuchen bei Säuglingen bzw. Kleinkindern ("wheezy infant syndrome");
Bronchiolitis, chronischer oder akuter Bronchokonstriktion, Obstruktion der kleinen Luftwege und Emphysem; obstruktiver oder entzündlicher Luftwegeerkrankungen eines beliebigen Typs, einer beliebigen Ätiologie oder Pathogenese, insbesondere eine obstruktive oder entzündliche Luftwegeerkrankung, die ein Mitglied ist, ausgewählt aus der Gruppe, bestehend aus chronischer eosinophiler Pneumonie, Schocklunge (ARDS), Exazerbation einer Hyperreaktivität der Luftwege als Folge einer anderen Wirkstofftherapie und Luftwegeerkrankung, die mit pulmonalem Hochdruck assoziiert ist,
Bronchitis-Formenkreis ausgewählt aus der Gruppe bestehend aus akuter laryngotrachealer Bronchitis, durch Erdnüsse ausgelöster Bronchitis ("arachidic bronchitis"), Bronchitis, catarrhalis, Kruppbronchitis, trockener Bronchitis, infektiöser asthmatischer Bronchitis, produktiver Bronchitis, Staphylococcus- oder Streptokokken-Bronchitis und Bläschenbronchitis,
akuter Lungenverletzung, Emphysem, posttraumatischer Lungeninsuffizienz;
Bronchiektasie eines beliebigen Typs, einer beliebigen Ätiologie oder Pathogenese, insbesondere Bronchiektasie-Formenkreis ausgewählt aus der Gruppe, bestehend aus zylinderförmiger Bronchiektasie, sackförmigen Bronchiektasen, fusiformer Bronchiektasie, Bronchiolektasie ("capillary bronchiectasis"), cystischer Bronchiektasie, trockener Bronchiektasie und follikulären Bronchiektasen.

2. Arzneimittel nach Anspruch 1 enthaltend Extrakte aus Thymian und Efeu oder Extrakte aus Thymian und Primel.

3. Verwendung eines Arzneimittels nach Anspruch 1 enthaltend Thymian und Efeu oder Thymian und Primel und ggfs. weitere Zusatz- und Hilfsstoffe.

4. Arzneimittel nach einem der vorhergehenden Ansprüche,
**dadurch gekennzeichnet, dass** es in einer galenischen Formulierung vorliegt, insbesondere eine orale Formulierung wie Dragees, Tabletten, Filmtabletten, Kapseln, flüssige Verdünnungen, insbesondere Tropfen, Säfte, Sirupe oder eine Formulierung zur topischen Anwendung wie Sprays, Salben, Emulsionen, Puder, Pulver, flüssige oder feste Präparate für die Inhalation, Kompressen, Tamponaden, Tonsillenpinsellösungen, Gurgellösungen oder Spüllösungen.

5. Pharmazeutische Zusammensetzung enthaltend ein Arzneimittel nach einem der vorhergehenden Ansprüche.

6. PDE-4 Inhibitor enthaltend Thymian und Efeu oder Extrakte aus Thymian und Primel oder Extrakte davon.
